# EUROPEAN PATENT APPLICATION

(11) **EP 1 501 037 A2**
(43) Date of publication of application: **26.01.2005**
(21) Application number: 04017063.1
(22) Date of filing: 20.07.2004
(51) Int. Cl.: G06F 19/00

(54) **An enhanced safety medication administration system**

(30) Priority: 25.07.2003 US 490330 P; 05.01.2004 US 751800
(71) Applicant: Siemens Medical Solutions Health Services Corporation, Malvern, PA 19355 (US)
(72) Inventor: Burkeen, Debra, 19460 Phoenixville, PA (US); Miller, Raymond F., 19352 Lincol University, PA (US)
(74) Representative: French, Clive Harry

(57) **Abstract**

A system provides dual independent medication administration clinical safety checks comprising a check upon medication ordering and a check substantially at the time of medication administration. A system checks safety of medication administration to a patient using an input processor for receiving, an identifier identifying a particular medication and an identifier identifying a particular patient. A first safety processor uses the received identifiers in checking for a clinical condition indicative of potentially impaired safety in administering the particular medication to the particular patient. The checking occurs substantially at the time of administering the particular medication to the particular patient. A display generator initiates generation of data representing at least one display image including a message alerting a user to the safety impairment. A second safety processor checks for a clinical condition indicative of potentially impaired safety in administering the particular medication to the particular patient using received information associated with an order prescribing the particular medication is to be administered to the particular patient.

## Description

### Field of the Invention

This invention concerns an information system supporting clinical safety checking of a scheduled administration of a medication to a patient.

### Background of the Invention

The administration of medication to a patient often does not run to schedule. The administration of continuous Intra-Venous (IV) infusions, for example, frequently run behind schedule, due to IV bag overfill, temporary stoppages to administer blood or other IV medications, rate changes, occlusions, etc. Further, between the time a medication is dispensed from a pharmacy and the time that the medication is actually administered to the patient, information concerning a patient condition may change (e.g., patient laboratory test values may become available or indicate medical condition changes). In the light of the new patient medical condition information, it may not be prudent to administer a scheduled medication to the patient. Existing medication information systems fail to provide comprehensive real time safety checking. A system according to invention principles addresses these deficiencies and associated problems.

### Summary of the Invention

The present invention accordingly provides apparatus and methods as defined in the appended claims.

For example, a medication administration safety checking system accommodates updates to patient medical record information occurring subsequent to initiation of an order for the medication to be administered to a patient. The system provides dual independent medication administration clinical safety checks comprising a check upon medication ordering and a check substantially at the time of medication administration and compares the results of the independent safety checks. A system checks safety of medication administration to a patient using an input processor for receiving, an identifier identifying a particular medication and an identifier identifying a particular patient. A first safety processor uses the received identifiers in checking for a clinical condition indicative of potentially impaired safety in administering the particular medication to the particular patient. The checking occurs substantially at the time of administering the particular medication to the particular patient. A display generator initiates generation of data representing at least one display image including a message alerting a user to the safety impairment. A second safety processor checks for a clinical condition indicative of potentially impaired safety in administering the particular medication to the particular patient using received information associated with an order prescribing the particular medication is to be administered to the particular patient.

In a feature of the invention, the first safety processor uses medical condition information of said particular patient acquired during an intervening time interval occurring between safety checks performed by said first and second safety processors.

### Brief Description of the Drawing

Figure 1 shows operational functions of a hospital, for example, including a clinical medication administration safety system, according to invention principles.
Figure 2 shows a user interface display image window indicating potential safety problems in administering a medication to a patient, according to invention principles.
Figure 3 shows a user interface display image window enabling a user to initiate an alert indicating a potential safety problem in administering a user initiated order for a medication to be administered to a patient, according to invention principles.
Figure 4 shows a user interface display image window enabling a user to view potential medication allergy conflicts, according to invention principles.
Figure 5 shows a user interface display image window enabling a user to view potential medication interaction conflicts, according to invention principles.
Figure 6 shows a user interface display image window enabling a user to view potential interference conflict between a medication and laboratory test results of a patient, according to invention principles.
Figure 7 shows a user interface display image window enabling a user to view potential IV infusion medication incompatibility conflicts, according to invention principles.
Figure 8 shows a user interface display image window enabling a user to view potential overdose conflicts, according to invention principles.
Figure 9 shows a user interface display image window enabling a user to view rules used in clinical safety checking, according to invention principles.
Figure 10 shows a user interface display image window enabling a user to record a clinical user intervention in response to detection of a potential clinical safety conflict prior to medication administration, according to invention principles.
Figure 11 shows a user interface display image used for configuring a medication administration clinical safety check performed at time of administering a medication to a patient, according to invention principles.
Figure 12 shows a flowchart of a process used by a medication management information system in performing medication administration clinical safety checks, according to invention principles.

### Detailed Description of Invention

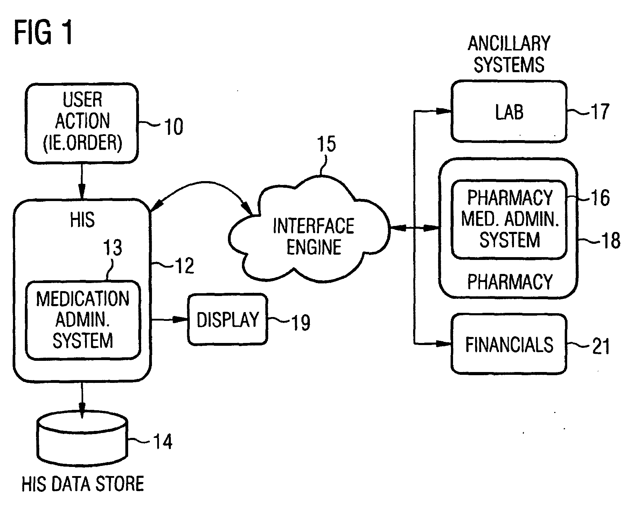

Figure 1 shows operational functions of a hospital, for example, including a clinical medication administration safety system. Healthcare Information System (HIS) 12 incorporates medication administration system 13 (such as an executable application) and responds to user actions 10, by bidirectionally communicating with external systems 17-21 through interface engine 15. In response to a user such as a clinician entering a medication order 10 for a patient, HIS 12 schedules delivery of the medication to a patient. The medication schedule for the patient is available for display to a user on PC 19 and data representing the medication schedule and other HIS and patient record data is stored in repository 14. Information is also entered by a user (such as a nurse or physician) recording actual time and date of administration of a medication to a patient via PC 19, for example.

System 13 advantageously checks for potential clinical and medicinal conflicts and warning conditions prior to medication administration at the point of administration of the medication, in response to user scanning of a patient bar code identifier and a medication label bar code, for example. Further, because the check is made at the time and point of medication administration (not just at the time of a physician placing the order for the medication), the system provides real time safety checking based on actual real data identifying medications and doses previously actually administered to a patient. The system improves safety checking in those instances where patient information is updated (such as in response to medication interaction tests or laboratory test results, which are updated as new laboratory results are generated) between the entry of a medication administration order (or since dispensing ordered medication) and the actual administration of the medication. Such a change in patient information means that the time at which medication is administered to a patient may be the first time that a clinical conflict (or other safety problem) can be detected by system 13. The system advantageously provides an additional medication administration safety check prior to medication administration. The system provides a check for potential conflicts prior to medication administration and also provides a clinical double-check. The safety of administering a medication to a patient is clinically checked prior to administration using two independent clinical systems, a Pharmacy application and a separate Medication Administration Check application, for example.

A Medication Administration Check application in system 13 provides the option of clinically checking safety of administering a particular medication to a particular patient prior to administering the medication. Checking occurs in response to a user scanning a medication label bar code and a patient identification bar code or in response to a healthcare worker (e.g., a nurse) entering data via PC 19 identifying actual time and date of administration of a medication and the medication itself. The system supports clinical checking of safety of administering a particular medication to a particular patient at the point of administration in addition to at the point of a physician entering an order to provide the medication to the patient. This significantly improves patient safety.

System 13 advantageously improves patient safety by automatically initiating on-line clinical medication safety checking in Pharmacy application 16 in pharmacy system 18 (e.g., checking for allergies, medication interactions, conflicts, dosage and other errors), substantially at the time of medication administration as well as at the time of ordering of the medication. For this purpose, a message identifying, a medication, patient and time of administration (e.g., derived in response to a scanned medication bar code at the point of administering the medication) is communicated by system 13 via interface engine 15 for use by pharmacy application 16 in system 18. Interface engine 15 may comprise a workflow processing application or other application supporting communication with external systems 17-21. A workflow as used herein comprises a sequence of tasks or operations that are scheduled for performance, or are being performed, by one or more entities including individuals, groups of individuals, or personnel assigned to perform particular functions or roles. External systems 17-21 comprise a laboratory system 17, pharmacy system 18 and a financial application (such as for patient service tracking and billing) 21, for example, but may also encompass a broader range of systems including any system with which HIS 12 performs a transaction or data exchange. Further Healthcare Information System (HIS) 12 may comprise other types of information system such as a Clinical Information System or Critical Care Information System or another Information system.

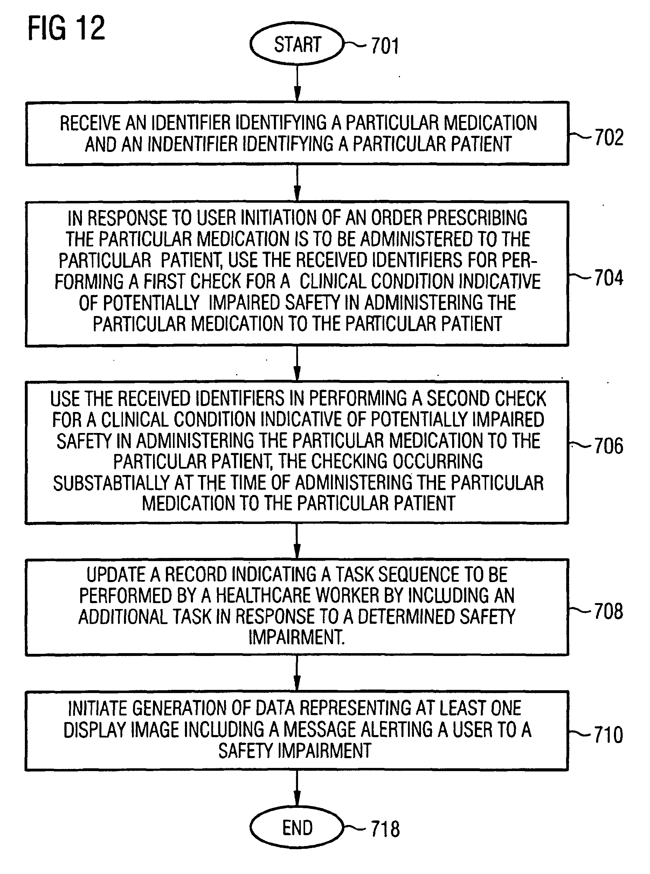

Figure 12 shows a flowchart of a process used by medication management information system 13 (in conjunction with pharmacy application 16) in performing medication administration clinical safety checks. In step 702 after the start at step 701, system 13 receives an identifier identifying a particular medication, an identifier identifying a particular patient and a time value indicating a time of administration of the particular medication. The identifiers and time value are received in response to user data entry via a data entry device used substantially at a place of administering the particular medication to the particular patient. The data entry device comprises a bar code scanner, for example. The time value, identifier identifying the particular medication and the identifier identifying the particular patient are derived from data comprising a bar code scan of a medication identifier and a bar code scan of a patient identifier. Alternatively, the data entry device may comprise a keyboard or other data entry device, for example.

In response to user initiation of an order prescribing the particular medication is to be administered to the particular patient, system 13 in step 704 uses the received time value and identifiers for performing a first check for a clinical condition indicative of potentially impaired safety in administering the particular medication to the particular patient. Pharmacy application 16 (operating in conjunction with system 13) in step 706 uses the received time value and identifiers in performing a second check for a clinical condition indicative of potentially impaired safety in administering the particular medication to the particular patient. The second safety check is performed substantially at the time of administering the particular medication to the particular patient. In alternative embodiments the clinical safety function of application 16 is embodied in system 13 or the clinical safety functions are embodied in application 16 or in one or more different applications located in any of the elements of Figure 1. A medication comprises, an IV infusion fluid, an orally administered medication, an injected medication or a medication applied externally to a patient's body, for example

Information associated with the first and second safety checks is communicated between system 13 and Pharmacy application 16. The first and second safety checks are independently performed by system 13 and Pharmacy application 16 respectively. The performed safety checks comprise, for example, (a) checking the particular medication is not already prescribed for administration to the particular patient, (b) checking the particular medication is not already prescribed as a component of another medication for administration to the particular patient, (c) checking for undesirable medication interaction with another medication prescribed for administration to the particular patient, (d) checking the particular medication is suitable for the particular patient based on age and paediatric related restrictions, and (e) checking the particular medication is suitable for the particular patient based on age and geriatric related restrictions.

The performed safety checks may also comprise, for example, (1) checking the particular medication is suitable for the particular patient based on laboratory test results of the particular patient, (2) checking the particular medication is suitable for the particular patient based on warnings associated with the particular medication, (3) checking the particular medication is suitable for the particular patient based on user determined rules, (4) checking the particular medication is suitable for administration to the particular patient based on whether the medication administration results in an overdose condition, (5) checking whether an existing under-dose condition affects administration of the particular medication to the particular patient, (6) checking the particular medication is suitable for administration to the particular patient based on cumulative medication dose administered to the particular patient and (7) checking the particular medication is suitable for administration to the particular patient in a particular IV infusion and is not incompatible with a different medication being administered to the particular patient as a component of the particular IV infusion.

A check by system 13 for a potential overdose conflict prior to medication administration involves summing a dose to be administered and doses (of this medication) that have been administered within the last 23 hours and 59 minutes to determine the daily total dose that is not to be exceeded in a 24-hour period. In contrast an order application associated clinical overdose check determines a daily total dose based on doses that are scheduled for administration. An overdose check may determine a dose to be administered exceeds either or both a single dose maximum and a 24-hour dose maximum. Separate overdose checks based on ordering and on administration provide independent safety checks. The criteria used to check for a potential overdose conflict is associated with a medication by its generic code sequence number. This number represents a generic formulation that is specific to one or more ingredients, medication strength, dosage form, and route of administration, but is the same for different manufacturers and package sizes. System 13 performs a safety check on medications that share the same generic code sequence number and are part of the same complex medication order or IV order by performing a group safety check for a potential overdose conflict (i.e., their doses are combined and it is the combined dose that is checked). For example, if a complex medication order combines a 500mg tablet and a 250mg tablet, to administer 750mg of acetaminophen, the combined dose of 750mg is checked for a potential overdose conflict.

System 13, in performing an overdose safety check, also identifies therapeutic duplicate medications taken by a patient and incorporates doses of any therapeutic duplicate in determining a cumulative dose for comparison with an overdose threshold. A therapeutic duplicate medication is a medication in the same therapeutic category as the medication to be administered to the patient (as an example, penicillin and tetracycline are in the same therapeutic category and are considered therapeutic duplicates). A system 13 overdose safety check compares a medication dose that has been dispensed from a pharmacy for a patient in addition to previous doses received by the patient with predetermined thresholds for the medication concerned. The thresholds include a warning-level cumulative dose threshold, a maximum cumulative dose threshold and a lifetime cumulative dose threshold.

A system 13 safety check compares a total dose a patient has received during his lifetime with a lifetime threshold and generates an alert message in response to a determination the threshold has been exceeded. The lifetime warning-level cumulative dose threshold is patient specific and based on body surface area (BSA). Further, a system 13 performed medication interaction safety check generates an indicator identifying interaction significance level including a contraindicated medication combination, severe interaction and moderate interaction, for example. A contraindicated medication combination indicates administration of a medication is contraindicated and should not be dispensed or administered to the same patient. A severe interaction indicates action is required to reduce risk of severe adverse interaction and a moderate interaction indicates an assessment of risk and further action are needed. System 13 enables a user to override a particular safety check result, except for a safety check result that requires mandatory intervention action. In addition system 13 initiates generation of a clinical check override report identifying safety checks overridden as well as the users responsible and associated times and dates. The report provides an audit trail of overridden clinical checks. The report is created for an overridden dosage, allergy or other clinical check and includes a summary that lists the overridden clinical checks.

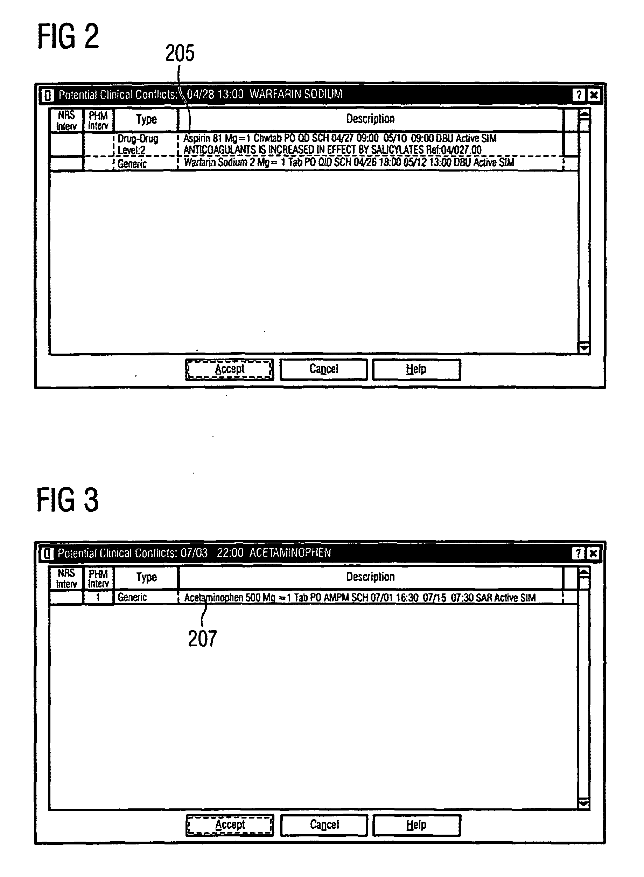

System 13 initiates generation of user interface display image menus 3-10 presenting medication conflicts, interactions and related characteristics. The user interface images of Figures 2 and 3 illustrate menus used for identification of potential safety problems such as potential clinical conflicts of a medication to be administered. Figures 2 and 3 illustrate potential clinical conflicts 205 and 207 respectively concerning particular medications, for example. The potential clinical conflicts alert window of Figures 2 and 3 are used to enable a user to review and address any potential clinical conflicts that are detected prior to administration of a medication. The window is also used to repeat a previously generated clinical conflict alert (e.g., generated by system 13 upon ordering of the medication) or is also used to provide an alert resulting from a clinical check substantially at the time and place of administration of a medication.

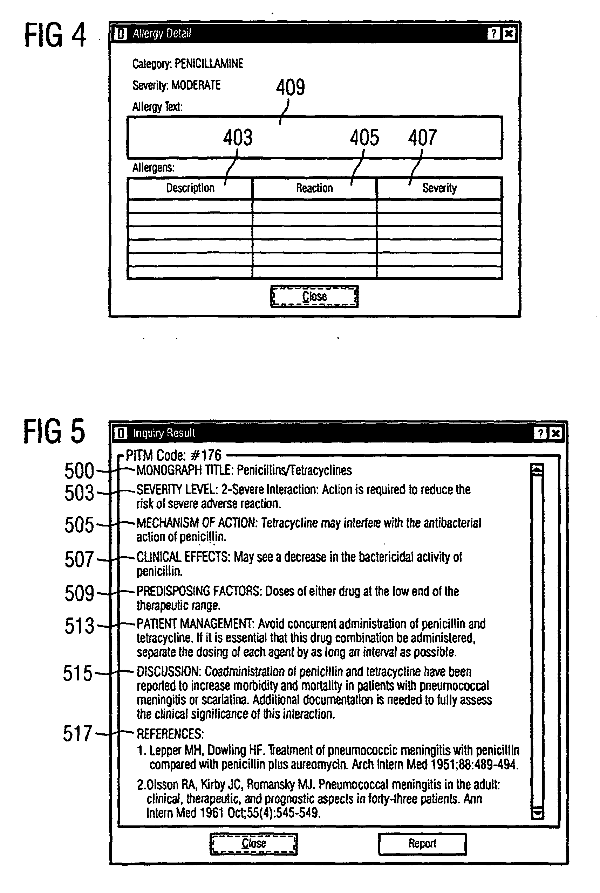

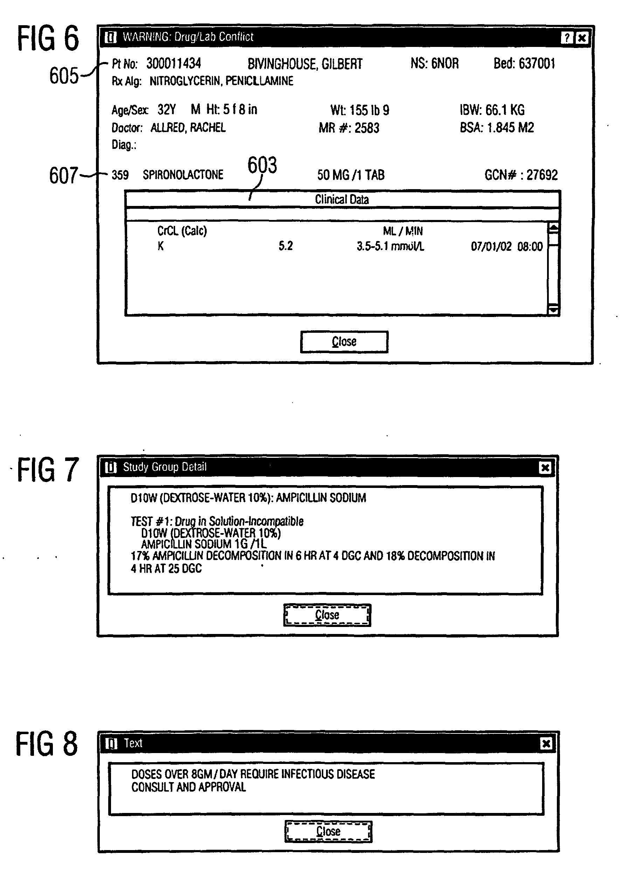

The image window of Figure 4 enables a user to view potential medication allergy conflicts. The window presents allergy definition text 409 as well as descriptive information 403, reactions 405 and severity indications 407. Similarly, the image window of Figure 5 enables a user to view potential medication interaction conflicts. These are characterized by title 500, severity level 503, mechanism of action 505, clinical effects 507, predisposing factors 509, patient management information 513, discussion 515 and references 517. Also the image window of Figure 6 enables a user to view potential interference conflicts between a medication and laboratory test results of a patient. Specifically, Figure 6 identifies a potential conflict between laboratory test results presented in panel 603 and a medication 607 for a particular patient 605.

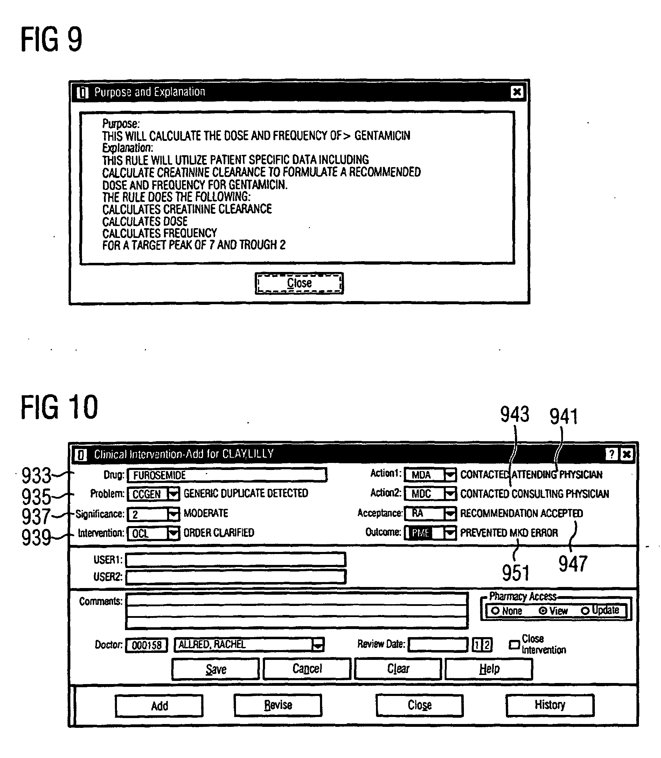

Figure 7 illustrates a user interface display image window enabling a user to view potential IV infusion medication incompatibility conflicts. Similarly, Figure 8 illustrates a user interface display image window enabling a user to view potential overdose conflicts. Figure 9 shows a user interface display image window enabling a user to view rules used in clinical safety checking. Specifically, Figure 9 shows a window explaining a rule for determining a dose and frequency of administration of a medication (Gentamicin) based on patient specific data. Figure 10 shows a user interface display image window enabling a user to record and document a clinical user intervention in response to detection of a potential clinical safety conflict prior to medication administration. A clinical intervention is a record of clinical activity, feedback, and comments concerning the administration of a medication. System 13 automatically generates a record of an intervention and also supports mandatory and optional manual recording of an intervention. A clinical intervention that is recorded during clinical checking is associated with a medication order that is being reviewed. An intervention can also be recorded independently of clinical checking and without association with a specific order.

Two clinical interventions may be recorded for each potential conflict: one during clinical checking via Medication Administration system 13 and one during clinical checking via Pharmacy application 16. A clinical intervention recorded during clinical checking is automatically assigned a sequential intervention number. In addition, the intervention number is combined with the associated order number for unique intervention identification, e.g., 3-1 represents the first intervention that has been recorded for Order Number 3. Further, predetermined codes and descriptions are used to record clinical interventions via system 13 (e.g., a problem code and a description of the reason for the intervention).

The Figure 10 image window enables a user to indicate a safety problem 935, a significance level 937 and an intervention action taken 939, for an identified medication 933. The window further allows a user to indicate actions taken 941 and 943 as well as to indicate whether a recommended action 947 was accepted by the patient and the outcome of the intervention 951. The image window of Figure 10 also provides four user-determinable fields that may be used to record information that is pertinent to an intervention. These fields include a Comments field, Pharmacy Access field as well as Doctor and Review Date fields. The Comments field enables a user to enter notes concerning an intervention and the Pharmacy Access field allows a user to determine a Level of access (such as no access, display and update access or read only access) to intervention data for Pharmacy Clinical Workstation users. The Doctor field enables a user to enter a name of a patient's Doctor and associated contact information. The Review Date field identifies when a pharmacist should review the user entered intervention information and the Close Intervention Indicator identifies that an intervention is closed.

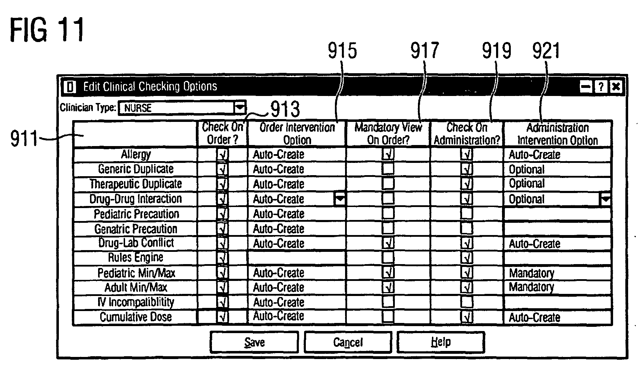

Figure 11 shows a user interface display image used for configuring a medication administration clinical safety check performed at time of administering a medication to a patient. The Figure 11 user interface image enables a Clinician of a particular Type to determine whether a particular clinical safety check is performed and whether a Clinical Intervention is required as a result of a conflict being identified by performing the particular clinical safety check. The Figure 11 user interface image enables a Clinician to determine whether a Clinical Intervention is mandatory, optional, or automatically initiated and whether a user needs to view identified conflict results. The Figure 11 user interface image also enables separate clinical safety checks to be configured to be performed both upon user order entry and upon medication administration. Specifically, the image enables clinical safety checks to be configured to be performed upon user entry of an order for administration of a particular medication to a patient and substantially upon administration of a medication.

Column 911 lists various safety checks that may be selected and configured. Columns 915 and 921 are used to associate manual intervention actions with individual safety checks. Column 915 enables a user to initiate an intervention action associated with an order (and with an order processing application) for administration of a medication to a patient. Column 921 enables a user to initiate an intervention action associated with pharmacy application 16 and administration of a medication to a patient. Column 913 enables a user to select individual safety checks to be performed in response to user entry of an order for administration of a medication to a patient in an order processing application in HIS 12 (Figure 1). Column 917 enables a user to select that it is mandatory to view results of a performed clinical safety check prior to the initiation of an order for a particular medication for a patient. Column 919 enables a user to select that a clinical safety check is performed, prior to and substantially near, or at, the time of administration of a particular medication to a patient.

A task manager within system 13 in step 708 updates a record indicating a task sequence to be performed by a healthcare worker by including an additional task in response to a safety impairment determined in the first or second safety check in steps 704 and 706. An additional task comprises a scheduled reminder to prompt a healthcare worker to do a follow-up patient assessment, for example. System 13 advantageously automatically initiates generation of a reminder to perform follow-up patient assessment and document the outcome, for example. Regulations and nursing best practice guidelines may require that a therapeutic effect, or patient outcome is documented after certain medications are administered. For example, a patient should be reassessed for pain relief 45 minutes after receiving an analgesic medication. In a manual environment, nurses need to remember to perform this task. As a result, a follow-up assessment is frequently omitted due to a high workload and a hectic work environment in the patient care area. System 13 automatically schedules a reminder on a task worklist to prompt a nurse to do a follow-up patient assessment. The determined scheduled reminder time takes into account the time duration involved in actually administering a medication to a patient.

System 13 provides a structured data entry form that prompts a nurse for data required in documenting an outcome, which, together with the automatic follow-up reminder scheduling significantly improves the accuracy and timeliness of documentation. This helps ensure compliance with practice guidelines and improves accuracy of data used in medication therapy management. The comprehensive structured data maintained by system 13 concerning medication administration times, doses, and effect or outcome, facilitate physician evaluation and adjustment of a patient medication regimen. Similar medication administration and effect data aggregated across multiple patients is used by system 13 in analyzing clinical effectiveness of standard medication dosing protocols.

System 13 in step 710 initiates generation of data representing one or more display images comprising a data entry form for prompting a healthcare worker to enter data associated with the follow-up patient assessment. The display images include a message alerting a user to a determined safety impairment. The process of Figure 12 terminates at step 718.

The user interface display images, systems and processes presented in Figures 1-12 are not exclusive. Other user interface and processing systems may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. A system according to invention principles may provides multiple independent clinical safety checks in other applications to support improved safety and management of resources.

## Claims

1. A system for checking safety of medication administration to a patient, comprising:
an input processor for receiving an identifier identifying a particular medication and an identifier identifying a particular patient;
a first safety processor for using said received identifiers in checking for a clinical condition indicative of potentially impaired safety in administering said particular medication to said particular patient, said checking occurring substantially at said time of administering said particular medication to said particular patient; and
a display generator for initiating generation of data representing at least one display image including a message alerting a user to a safety impairment.

2. A system according to claim 1, wherein
said input processor receives said identifier identifying said particular medication and said identifier identifying said particular patient via a data entry device used substantially at a place of administering said particular medication to said particular patient and
said data entry device comprises a bar code scanner and said identifier identifying said particular medication and said identifier identifying said particular patient are derived from data comprising a bar code scan of a medication identifier and a bar code scan of a patient identifier.

3. A system according to claim 1 or claim 2, wherein
said first safety processor uses a time value indicating a time of medication administration received by said input processor in checking for a clinical condition indicative of potentially impaired safety.

4. A system according to any preceding claim, including
a second safety processor for checking for a clinical condition indicative of potentially impaired safety in administering said particular medication to said particular patient using an identifier identifying said particular medication and an identifier identifying said particular patient derived from received information associated with an order prescribing said particular medication is to be administered to said particular patient.

5. A system according to claim 4, wherein
said first and said second safety processors independently check for said clinical condition indicative of potentially impaired safety in administering said particular medication to said particular patient and
a result of said clinical check performed by said first safety processor is communicated to said second safety processor.

6. A system according to claim 4, wherein
said first and second safety processors exchange data representing results of clinical checks and
a check for a clinical condition indicative of potentially impaired safety uses medical condition information of said particular patient acquired during an intervening time interval occurring between safety checks performed by said first and second safety processors.

7. A system according to any of claims 4-6, wherein
said checking for said clinical condition indicative of potentially impaired safety in administering said particular medication to said particular patient, comprises at least one of, (a) checking said particular medication is not already prescribed for administration to said particular patient, (b) checking said particular medication is not already prescribed as a component of another medication for administration to said particular patient, (c) checking for undesirable medication interaction with another medication prescribed for administration to said particular patient, (d) checking said particular medication is suitable for said particular patient based on age and paediatric related restrictions, and (e) checking said particular medication is suitable for said particular patient based on age and geriatric related restrictions.

8. A system according to any of claims 4-7, wherein
said checking for said clinical condition indicative of potentially impaired safety in administering said particular medication to said particular patient, comprises at least one of, (a) checking said particular medication is suitable for said particular patient based on laboratory test results of said particular patient, (b) checking said particular medication is suitable for said particular patient based on warnings associated with said particular medication, (c) checking said particular medication is suitable for said particular patient based on user determined rules, (d) checking said particular medication is suitable for administration to said particular patient based on whether said medication administration results in an overdose condition, (e) checking whether an existing under-dose condition affects administration of said particular medication to said particular patient, (f) checking said particular medication is suitable for administration to said particular patient based on cumulative medication dose administered to said particular patient and (g) checking said particular medication is suitable for administration to said particular patient in a particular IV infusion and is not incompatible with a different medication being administered to said particular patient as a component of said particular IV infusion.

9. A system according to any preceding claim, wherein
said medication comprises at least one of, (a) an IV infusion fluid, (b) an orally administered medication, (c) an injected medication and (d) a medication applied externally to a patient's body and including
a task manager for updating a record indicating a task sequence to be performed by a healthcare worker by including an additional task in response to a determined safety impairment wherein
said additional task comprises a scheduled reminder to prompt a healthcare worker to do a follow-up patient assessment.

10. A system according.to claim 9, wherein
said display generator initiates generation of data representing a display image comprising a data entry form for prompting a healthcare worker to enter data associated with said follow-up patient assessment.

11. A system for checking safety of medication administration to a patient, comprising:
an input processor for receiving an identifier identifying a particular medication and an identifier identifying a particular patient;
a first safety processor for using said received identifiers for performing a first check for a clinical condition indicative of potentially impaired safety in administering said particular medication to said particular patient, said first check occurring in response to user initiation of an order prescribing said particular medication is to be administered to said particular patient;
a second safety processor for using said received identifiers in performing a second check for a clinical condition indicative of potentially impaired safety in administering said particular medication to said particular patient using medical condition information of said particular patient acquired during an intervening time interval occurring between said first and second checks, said second check occurring substantially at said time of administering said particular medication to said particular patient; and
a display generator for initiating generation of data representing at least one display image including a message alerting a user to a safety impairment.

12. A system according to claim 11, wherein
at least one of, (a) information associated with said first check is communicated from said first safety processor to said second safety processor and (b) information associated with said second check is communicated from said second safety processor to said first safety processor and
said data representing said at least one display image includes said information associated with said first check or second safety check.

13. A system according to claim 11 or claim 12, wherein
said first and said second safety processors independently check for said clinical condition indicative of potentially impaired safety in administering said particular medication to said particular patient at different times and
said input processor receives said identifier identifying said particular medication and said identifier identifying said particular patient via a data entry device used substantially at a place of administering said particular medication to said particular patient.

14. A system for checking safety of medication administration to a patient, comprising:
an input processor for receiving an identifier identifying a particular medication and an identifier identifying a particular patient;
a first safety processor for using said received identifiers for performing a first check for a clinical condition indicative of potentially impaired safety in administering said particular medication to said particular patient, said first check occurring in response to user initiation of an order prescribing said particular medication is to be administered to said particular patient;
a second safety processor for using said received identifiers in performing a second check for a clinical condition indicative of potentially impaired safety in administering said particular medication to said particular patient using medical condition information of said particular patient acquired during an intervening time interval occurring between said first and second checks, said second check occurring substantially at said time of administering said particular medication to said particular patient; and
a task manager for updating a record indicating a task sequence to be performed by a healthcare worker by including an additional task in response to a determined safety impairment.

15. A method for checking safety of medication administration to a patient, comprising the activities of:
receiving an identifier identifying a particular medication and an identifier identifying a particular patient;
using said received identifiers in checking for a clinical condition indicative of potentially impaired safety in administering said particular medication to said particular patient, said checking occurring substantially at said time of administering said particular medication to said particular patient; and
initiating generation of data representing at least one display image including a message alerting a user to a safety impairment.

16. A method for checking safety of medication administration to a patient, comprising the activities of:
receiving an identifier identifying a particular medication and an identifier identifying a particular patient;
using said received identifiers for performing a first check for a clinical condition indicative of potentially impaired safety in administering said particular medication to said particular patient, said first check occurring in response to user initiation of an order prescribing said particular medication is to be administered to said particular patient;
employing said received identifiers in performing a second check for a clinical condition indicative of potentially impaired safety in administering said particular medication to said particular patient using medical condition information of said particular patient acquired during an intervening time interval occurring between said first and second checks, said second check occurring substantially at said time of administering said particular medication to said particular patient; and
initiating generation of data representing at least one display image including a message alerting a user to a safety impairment.

17. A method according to claim 16, including the activity of
updating a record indicating a task sequence to be performed by a healthcare worker by including an additional task in response to a determined safety impairment.
